# EUROPEAN PATENT APPLICATION

(11) **EP 4 372 056 A1**
(43) Date of publication of application: **22.05.2024**
(21) Application number: 22841870.3
(22) Date of filing: 17.06.2022
(51) Int. Cl.: C09B 11/28, C07B 61/00

(54) **XANTHENE COMPOUND AND METHOD FOR PRODUCING SAME**

(30) Priority: 13.07.2021 JP 2021116004
(71) Applicant: TAIYO HOLDINGS CO., LTD., Saitama 355-0222 (JP); Taiyo Fine Chemicals Co., Ltd., Nihonmatsu-shi, Fukushima, 964-0982 (JP)
(72) Inventor: TAYAMA Tomoya, Saitama-shi, Saitama 338-0835 (JP); YAMAOKA Jun, Saitama-shi, Saitama 338-0835 (JP); SAHARA Go, Hiki-gun, Saitama 355-0222 (JP)
(74) Representative: Hoffmann Eitle
(86) International application number: PCT/JP2022/024416
(87) International publication number: WO 2023/286526

(57) **Abstract**

A xanthene compound with excellent heat resistance is provided together with a method for producing the same. The xanthene compound is represented by the formula (1).

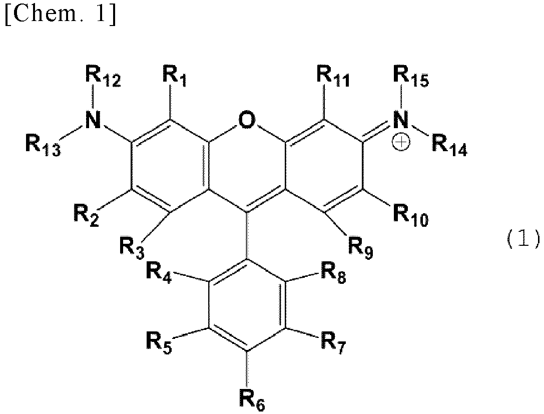

## Description

### TECHNICAL FIELD

The present disclosure relates to a xanthene compound and a method for producing the same.

### BACKGROUND

Xanthene compounds are important compounds as pigments due to their excellent coloration properties and are being applied in a wide variety of fields. One problem associated with such xanthene compounds is their lower heat resistance compared to pigments in different structures. To overcome this problem, various studies have been conducted.

For instance, it is proposed to incorporate an intermolecular salt-type xanthene compound with a certain structure into a coloring-photosensitive composition (PTL 1).

### CITATION LIST

### Patent Literature

JP2015060154A

### SUMMARY

### (Technical Problem)

However, although the xanthene compound in PTL 1 was developed with heat resistance as one of the problems, the recent demand for increased heat resistance has become more stringent to expand the fields and applications where xanthene pigments are utilized.

An object of the present disclosure is to provide a xanthene compound with excellent heat resistance, together with a method for producing the same.

### (Solution to Problem)

As a result of diligent studies, the present inventors have discovered that the aforementioned problem can be solved by an intramolecular salt-type xanthene compound that has a cationic xanthene skeleton and has at least one fluorine or chlorine atom and at least one anionic group introduced into a benzene ring bonded to the pyran ring of the cationic xanthene skeleton, thereby completing the present disclosure.

The subject matter of the present disclosure is as follows.
[1] A xanthene compound represented by the following formula (1): 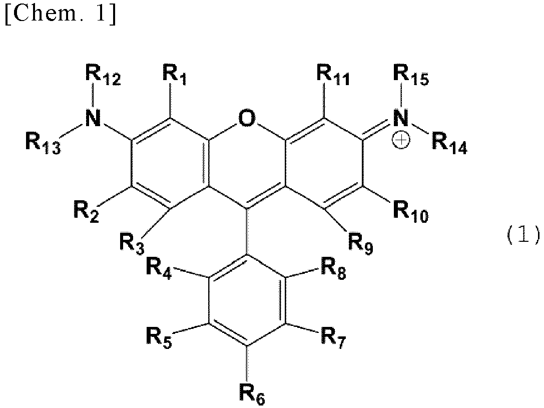 (where:
   R₁ to R₃ and R₉ to R₁₁ are each independently a hydrogen atom, a halogen atom, a cyano group, a nitro group, a hydroxy group, a sulfone group, an alkyl group having 1 to 8 carbon atoms, a cycloalkyl group having 3 to 8 carbon atoms, an alkoxy group having 1 to 8 carbon atoms, an acyl group having 1 to 21 carbon atoms, an aryl group having 6 to 20 carbon atoms, or a five- to eight-membered heteroaryl group having 1 to 3 heteroatoms selected from nitrogen, oxygen, and sulfur atoms, wherein the alkyl group, the cycloalkyl group, and the alkoxy group may be substituted with a halogen atom or an aryl group having 6 to 20 carbon atoms, and the aryl group and the heteroaryl group may be substituted with a halogen atom, a cyano group, a nitro group, or a hydroxy group,
   R₄ to R₈ are each independently a hydrogen atom, an organic group, a fluorine atom, a chlorine atom, or an anionic group, with the proviso that at least one of R₄ to R₈ is a fluorine or chlorine atom and at least one of R₄ to R₈ is an anionic group,
   R₁₂ to R₁₅ are each independently a hydrogen atom, an alkyl group having 1 to 8 carbon atoms, an aryl group having 6 to 20 carbon atoms, or a five- to eight-membered heteroaryl group having 1 to 3 heteroatoms selected from nitrogen, oxygen, and sulfur atoms, wherein the alkyl group may be substituted with an aryl group having 6 to 20 carbon atoms,
   R₁ and R₁₂, R₂ and R₁₃, R₁₁ and R₁₅, and R₁₀ and R₁₄ may each be linked to form a six-membered ring, and
   R₁₂ and R₁₃, and R₁₄ and R₁₅ may each be linked to form a three- to six-membered ring).
[2] The xanthene compound according to [1], wherein the anionic group is -SO₃⁻ or -SO₂N⁻SO₂R_{f} (where Rf is an alkyl group having 1 to 4 carbon atoms in which at least two hydrogen atoms are substituted with fluorine atoms).
[3] The xanthene compound according to [1] or [2], wherein at least one of R₄ and R₈ is a fluorine or chlorine atom.
[4] The xanthene compound according to [3], wherein both of R₄ and R₈ are fluorine or chlorine atoms.
[5] A method for producing the xanthene compound of the formula (1) according to any of [1] to [4], the method comprising:
   reacting, in the presence of an acid, a compound represented by the formula (10): 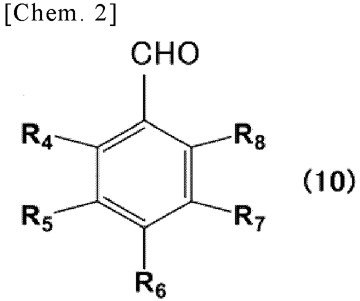 (where R₄ to R₈ are as defined in the formula (1) above), with compounds represented by the formulae (11) and (12): 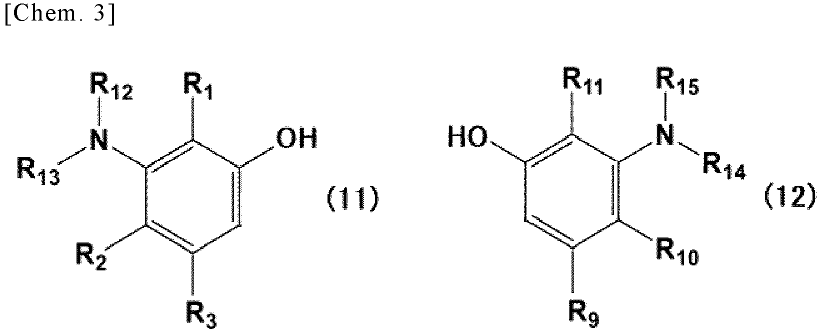 (where:
   R₁ to R₃ and R₁₂ to R₁₃ are as defined in the formula (1) above, and
   R₉ to R₁₁ and R₁₄ to R₁₅ are as defined in the formula (1) above); and
   oxidizing a resultant compound represented by the formula (13): 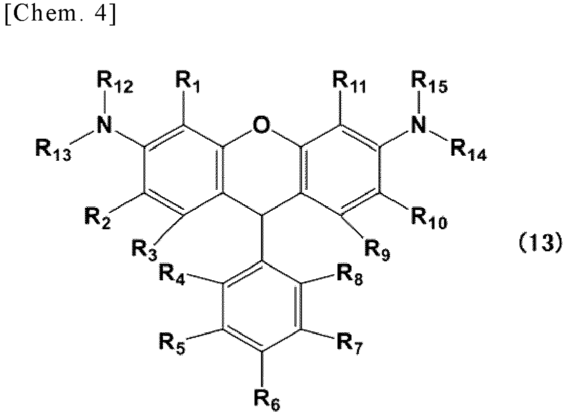 (wherein R₁ to R₁₅ are as defined in the formula (1) above).

### (Advantageous Effect)

According to the present disclosure, a xanthene compound with excellent heat resistance is provided together with a method for producing the same.

### DETAILED DESCRIPTION

In the following, the present disclosure will be described in detail.

### <Xanthene compound>

A xanthene compound of the present disclosure is represented by the following formula (1): 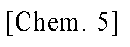 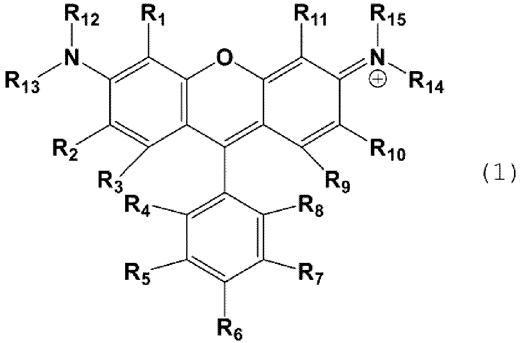 (where:
R₁ to R₃ and R₉ to R₁₁ are each independently a hydrogen atom, a halogen atom, a cyano group, a nitro group, a hydroxy group, a sulfone group, an alkyl group having 1 to 8 carbon atoms, a cycloalkyl group having 3 to 8 carbon atoms, an alkoxy group having 1 to 8 carbon atoms, an acyl group having 1 to 21 carbon atoms, an aryl group having 6 to 20 carbon atoms, or a five- to eight-membered heteroaryl group having 1 to 3 heteroatoms selected from nitrogen, oxygen, and sulfur atoms, wherein the alkyl group, the cycloalkyl group, and the alkoxy group may be substituted with a halogen atom or an aryl group having 6 to 20 carbon atoms, and the aryl group, the heteroaryl group, and the acyl group may be substituted with a halogen atom, a cyano group, a nitro group, or a hydroxy group,
R₄ to R₈ are each independently a hydrogen atom, an organic group, a fluorine atom, a chlorine atom, or an anionic group, with the proviso that at least one of R₄ to R₈ is a fluorine or chlorine atom and at least one of R₄ to R₈ is an anionic group,
R₁₂ to R₁₅ are each independently a hydrogen atom, an alkyl group having 1 to 8 carbon atoms, an aryl group having 6 to 20 carbon atoms, or a five- to eight-membered heteroaryl group having 1 to 3 heteroatoms selected from nitrogen, oxygen, and sulfur atoms, wherein the alkyl group may be substituted with an aryl group having 6 to 20 carbon atoms,
R₁ and R₁₂, R₂ and R₁₃, R₁₁ and R₁₅, and R₁₀ and R₁₄ may each be linked to form a six-membered ring, and
R₁₂ and R₁₃, and R₁₄ and R₁₅ may each be linked to form a three- to six-membered ring).

With regards to R₁ to R₃ and R₉ to R₁₁, examples of the halogen atom include fluorine, chlorine, bromine, and iodine atoms.

The alkyl group having 1 to 8 carbon atoms may be linear or branched, and examples thereof include methyl, ethyl, propyl, isopropyl, *n*-butyl, isobutyl, *sec*-butyl, *tert*-butyl, pentyl, hexyl, heptyl, and octyl.

Examples of the cycloalkyl group having 3 to 8 carbon atoms include cyclopropyl, cyclobutyl, cyclopentyl, and cyclohexyl.

With regards to the alkoxy group having 1 to 8 carbon atoms, the alkyl portion may be linear or branched. For the alkyl portion, the above explanations for an alkyl group are applied. Specifically, examples of the alkoxy group include methoxy, ethoxy, propyloxy, isopropyloxy, *n*-butyloxy, isobutyloxy, *sec*-butyloxy, *tert*-butyloxy, pentyloxy, hexyloxy, heptyloxy, and octyloxy.

Examples of the aryl group having 6 to 20 carbon atoms include phenyl, *o*-tolyl, *m*-tolyl, *p*-tolyl, 2,3-xylyl, 2,4-xylyl, 2,6-xylyl, 2-methyl-6-ethylphenyl, 2,6-diethylphenyl, 2,6-diisopropylphenyl, 3,4-xylyl, 3,5-xylyl, naphthyl, and anthracenyl.

The acyl group is a monovalent group represented by RC(=O)-, where R is a hydrogen atom, an alkyl group, or an aryl group. For the alkyl or aryl groups, the descriptions for the alkyl group having 1 to 8 carbon atoms or the aryl group having 6 to 20 carbon atoms are applied. Examples of the acyl group include acetyl and benzoyl groups.

The five- to eight-membered heteroaryl group having 1 to 3 heteroatoms selected from nitrogen, oxygen, and sulfur atoms may be a monocyclic ring or a condensed ring, and examples thereof include imidazolyl, benzimidazolyl, pyrazolyl, pyrrolyl, indazolyl, indolyl, triazolyl, furyl, oxazolyl, isooxazolyl, thienyl, thiazolyl, and isothiazolyl.

The alkyl group, the cycloalkyl group, and the alkoxy group described above may be substituted with a halogen atom or an aryl group having 6 to 20 carbon atoms, such as benzyl, for example.

The aryl group, the heteroaryl group, and the acyl group described above may be substituted with a halogen atom, a cyano group, a nitro group, or a hydroxy group.

R₁ and R₁₁ may be the same or different, preferably the same, and may be a hydrogen atom, for example.

R₂ and R₁₀ may be the same or different, preferably the same, and may be a hydrogen atom, for example.

R₃ and R₉ may be the same or different, preferably the same, and may be a hydrogen atom, for example.

Particularly, R₁ and R₁₁, R₂ and R₁₀, and R₃ and R₉ are preferably all the same.

With regards to R₄ to R₈, the anionic group is not particularly limited and is any group in the form of anion. Examples include groups in the form of monovalent anion, such as sulfonic acid-based, sulfonimide-based, carboxylic acid-based monovalent anions. Of these, preferred are groups in the form of sulfonic acid-based and sulfonimide-based monovalent anions because the influences on coloration caused by changes of surrounding environment (e.g., changes of pH) are suppressed.

Specific examples of the anionic group include, for example, -SO₃⁻, -SO₂N⁻SO₂R_{f} (where Rf is an alkyl group having 1 to 4 carbon atoms in which at least two hydrogen atoms are substituted with fluorine atoms), and -COO⁻, and -SO₃⁻ and -SO₂N⁻SO₂R_{f} are preferred. When two or more anionic groups are present, at least one anionic group forms an intramolecular salt, while other anionic groups may be in the form of salt, such as alkali metal salts (e.g., Na salt, K salt, etc.) and ammonium salts.

At least one of R₄ to R₈ is a fluorine or chlorine atom and at least one of R₄ to R₈ is an anionic group. When R₄ to R₈ other than a fluorine or chlorine atom and an anionic group are present, they are a hydrogen atom or an organic group. The organic group is not in ionic form. Examples of the organic group include sulfonic acid- or sulfonate ester-based groups, sulfonamide-based groups, sulfonylimide-based groups, carboxylic acid- or carboxylate ester-based groups, and carboxylic acid amide-based groups. However, these are not in ionic form.

Specific examples of the organic group include -SO₃R, -SO₂NR₂, -SO₂NHSO₂R, -COOR, and -CONR₂ (where R is hydrogen, an alkyl group having 1 to 4 carbon atoms which may be substituted with a fluorine atom).

At least one of R₄ to R₈ is a fluorine or chlorine atom, and at least one of R₄ and R₈ may be a fluorine or chlorine atom and both of R₄ and R₈ may be fluorine or chlorine atoms.

In view of solubility in organic solvents, one is preferably an anionic group, and any of R₅ to R₇ is preferably an anionic group.

With regards to R₁₂ to R₁₅, for the alkyl group having 1 to 8 carbon atoms, the aryl group having 6 to 20 carbon atoms, and the five- to eight-membered heteroaryl group having 1 to 3 heteroatoms selected from nitrogen, oxygen, and sulfur atoms, the corresponding descriptions in the above descriptions for R₁ to R₃ and R₉ to R₁₁ are applied. Also for the alkyl group substituted with an aryl group having 6 to 20 carbon atoms, the corresponding descriptions in the descriptions for R₁ to R₃ and R₉ to R₁₁ are applied.

R₁ and R₁₂, R₂ and R₁₃, R₁₁ and R₁₅, and R₁₀ and R₁₄ may each be linked to form a 6-membered ring. Examples of the 6-membered ring include a piperidine ring, a pyridine ring, a pyrimidine ring, a quinoline ring, and an isoquinoline ring. These rings may be fused with another ring or substituted.

R₁₂ and R₁₃, and R₁₄ and R₁₅ may each be linked to form a three- to six-membered ring. Examples of the three- to six-membered ring include a piperidine ring, a piperazine ring, a pyrrolidine ring, a morpholine ring, a thiomorpholine ring, a pyridine ring, a pyrazine ring, a pyrimidine ring, a quinoline ring, an isoquinoline ring, an imidazole ring, an oxazole ring, an imidazolidine ring, a pyrazolidine ring, an isooxazolidine ring, an isothiazolidine ring. These rings may be fused with another ring or substituted.

R₁₂ and R₁₃ may be the same or different.

R₁₄ and R₁₅ may be the same or different.

The combination of R₁₂ and R₁₃ and the combination of R₁₄ and R₁₅ may be the same or different.

An example of the xanthene compound represented by the formula (1) is a compound represented by the following formula (1'): 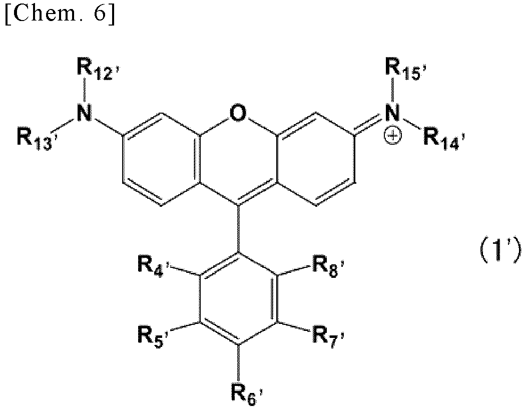 (where:
R₄, and R₈, are a hydrogen atom, a fluorine atom, or a chlorine atoms, at least one of which is a fluorine atom or a chlorine atom, preferably both of which are fluorine or chlorine atoms,
R_{5'} to R_{7'} are a hydrogen atom or an anionic group, with the proviso that at least one of R_{5'} to R_{7'} is an anionic group, preferably one of R_{5'} to R_{7'} is an anionic group and the rest are hydrogen atoms, and
R_{12'} to R_{15'} are an alkyl group having 1 to 8 carbon atoms or an aryl group having 6 to 20 carbon atoms, wherein the alkyl group is substituted with an aryl group having 6 to 20 carbon atoms).

The xanthene compound of the present disclosure has a maximum absorption wavelength in the range of 520 to 580 nm when the compound is dissolved in an organic solvent to prepare a solution and the UV-visible absorption spectrum is measured using the solution at about room temperature (about 25 °C).

The xanthene compound of the present disclosure has excellent heat resistance. For example, when the xanthene compound of the present disclosure is mixed with a resin solution and the resultant is coated as a film on a substrate and is subjected to a heat treatment, the color difference of the chromatic values between before and after the heat treatment (ΔE*_{ab}) is minimized. For instance, the color difference ΔE*_{ab} before and after the heat treatment at 230 °C for 1 hour can be maintained to less than 6.0.

### <Method for producing xanthene compound>

The xanthene compound of the present disclosure can be produced by a production method including the following steps.

### <Step A>

In the step A, in the presence of an acid, a compound represented by the formula (10): 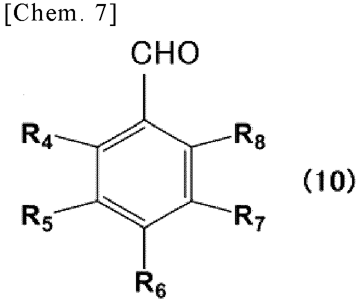 (where R₄ to R₈ are as defined in the formula (1) above), is reacted with compounds represented by the formulae (11) and (12): 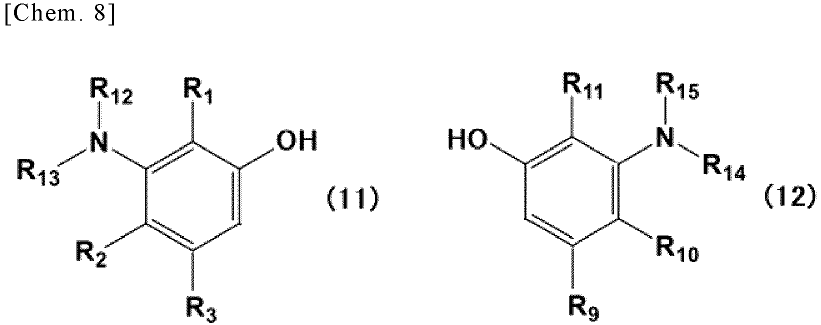 (where:
R₁ to R₃ and R₁₂ to R₁₃ are as defined in the formula (1) above, and
R₉ to R₁₁, R₁₄ to R₁₅ are as defined in the formula (1) above).

The total amount of the compounds represented by the formulae (11) and (12) may be from 2 to 6 mol per 1 mol of the compound represented by the formula (10). It is preferable to use the compound represented by the formula (11) and the compound represented by the formula (12) in equimolar amounts..

The reaction is carried out in the presence of an acid. The acid may be an organic acid (e.g., acetic acid, *p*-toluenesulfonic acid, etc.) or an inorganic acid (e.g., sulfuric acid, hydrochloric acid, etc.), but inorganic acids are preferred, with sulfuric acid being more preferred.

The reaction may be caused to take place in a solvent. Examples of the solvent include, for example, well-known and widely-used organic solvents (e.g., acetic acid) and inorganic solvents (e.g., water).

The reaction may be caused to take place from 100 to 160 °C, preferably from 110 to 140 °C.

As a result, a xanthene skeleton is formed and a compound represented by the formula (13) is obtained: 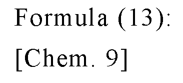 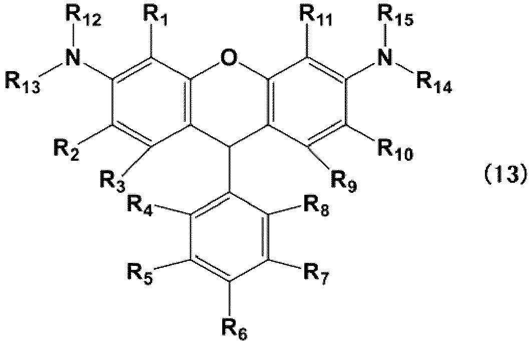 (in the formula, R₁ to R₁₅ are as defined in the formula (1) above).

The resulting reaction solution containing the compound of the formula (13) may be dripped into water to induce crystallization. Subsequently, filtration, washing, and drying may be performed. Separation and purification by column chromatography may be performed.

The compound of the formula (13) may be produced by reacting the compound of the formula (10) with a compound of the formula (11) and then further reacting with a compound of the formula (12). In this case, the amount of the compound of the formula (11) is preferably from 1.0 to 1.2 mol, and the amount of the compound of the formula (12) is preferably from 1.0 to 2.0 mol, per 1 mol of the compound of the formula (10).

The compound of the formula (10) may be reacted with the compound of the formula (12) first and is then reacted with the compound of the formula (11).

This production method is advantageous when the target compound is a compound in which the structures of the two rings fused to the pyran ring of the xanthene skeleton are different.

### <Step B>

In the step B, the compound represented by the formula (13) is oxidized by an oxidizing agent to obtain the compound represented by the formula (1).

Examples of the oxidizing agent include iron(III) chloride and p-chloranil, and iron(III) chloride is preferred.

The reaction may be caused to take place in a solvent. Examples of the solvent include, for example, water, hydrochloric acid, and sulfuric acid, and a hydrochloric acid solution is preferred.

The reaction may be caused to take place from 20 to 100 °C, preferably from 60 to 100 °C.

When the anionic group is a -SO₃⁻ group, it may be converted to -SO₂N⁻SO₂R_{f} by reacting R_{f}SO₂NH₂. Here, R_{f} is as defined in the formula (1).

The resulting compound of the formula (1) may be purified. Purification can be achieved, for example, by column chromatography, washing with a solvent, and recrystallization.

The xanthene compound of the present disclosure can be used in the production of a variety of paints, water-based or oil-based inks, or the like., and is also useful as a functional dye for recording materials or the like. The xanthene compound of the present disclosure can be used in combination with various additives, such as other organic dye materials, inorganic dye materials, thermoplastic resins, thermosetting resins, light-curable resins, heavy metal deactivating agents, metal soaps (e.g., alkali metal, alkaline earth metal, or zinc metal soaps), hydrotalcite, surfactants (e.g., nonionic, cationic, anionic, or amphoteric surfactants), antistatic agents, flame retardants (e.g., halogen-based flame retardants, phosphorus-based flame retardants, or metal oxide-based flame retardants), lubricants (e.g., ethylene bisalkylamide), antioxidizing agents, UV absorbers, processing aids, and fillers, within a range that does not inhibit the effects of the present disclosure.

### EXAMPLES

Although the present disclosure will be described in more detail below by way of examples, the present disclosure is not limited thereto.

Xanthene compounds were evaluated as follows.

### <Heat resistance>

The compounds of examples and comparative examples were each dissolved in 2 g of *N*-methylpyrrolidone. The solution was prepared by adding 0.986 g of a resin (CYCLOMER P (ACA) Z320 manufactured by Daicel Corporation) and 0.014 g of a 4-wt% solution of a leveling agent (BYK-333 manufactured by BYK-Chemie) in propylene glycol monomethyl ether acetate (PMA). The resulting solution was applied to a glass plate with a thickness of 2 µm and pre-baked on a hot plate at 100 °C for 3 minutes. The chromatic values (L*a*b*) of the obtained film were measured by transmitted light using a spectrometer (CM-5 manufactured by Konica Minolta, Inc.).

The film was then held in a thermostatic oven at 230 °C for certain time durations, and the chromatic values were measured in the same manner for the film after being held. The color differences of the chromatic values before and after being held at 230 °C (ΔE*_{ab}) were determined.

The amounts of the compounds used in the preparation of the solutions were adjusted so that the absorbance was equal. Specifically, the solutions were prepared with 0.036 g of Compound 1, 0.044 g of Compound 2, 0.052 g of Compound 3, 0.044 g of Compound 4, and 0.060 g of Comparative Compound 1.

### <Example 1>

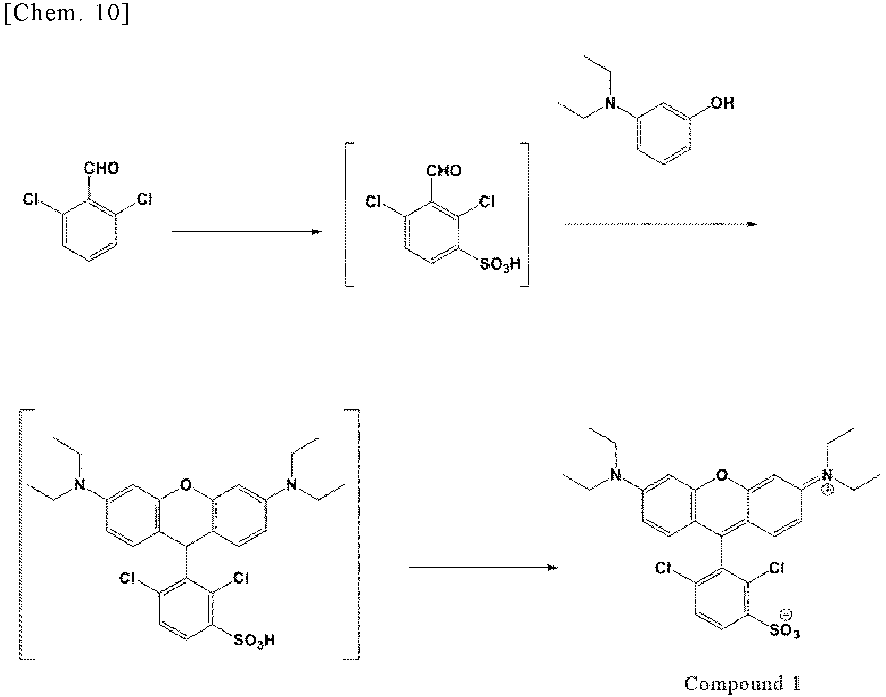

After 0.04 mol of 2,6-dichlorobenzaldehyde was mixed with 0.10 mol of 20% oleum, the mixture was stirred at 70 °C for 20 hours. After being cooled to room temperature, the reaction solution was dropped into water and washed with toluene. The resulting aqueous layer was mixed with 0.08 mol of diethylaminophenol and 0.50 mol of sulfuric acid. After being held at 90 °C for 17 hours, the reaction mixture was stirred at 120 °C for 1 hour to cause a reaction to take place. After being cooled to room temperature, the reaction solution was slowly added dropwise to an aqueous sodium hydroxide solution, and the resulting solid (33 g) obtained through filtration was mixed with 0.18 mol of dilute hydrochloric acid and 0.07 mol of a 35-mass% iron chloride solution. The mixture was stirred at 80 °C for 3 hours. After cooling to room temperature, chloroform was added. The aqueous layer was drained, and the resultant was washed with water and neutralized with sodium bicarbonate solution. The solvent was removed from the resulting organic layer. The residue was purified by silica gel column chromatography (developing solvent: chloroform:methanol = 95:5) to yield 1.7 g (0.003 mol) of Compound 1.
¹H-NMR (400 MHz, acetone-d6) : δ (ppm) = 8.35 (1H), 7.64 (1H), 7.23 (4H), 7.46 (2H), 7.01 (2H), 3.81 (8H), 1.36 (12H)

### <Example 2>

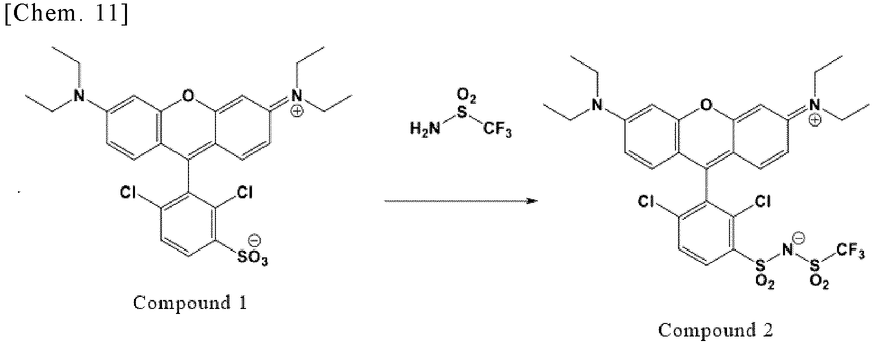

After 0.001 mol of Compound 1 was mixed with 0.50 mol of acetonitrile, 0.003 mol of phosphoryl chloride was added at 70 °C and the mixture was stirred for 2 hours. After cooling to 5 °C, 0.003 mol of trifluoromethanesulfonamide and 0.02 mol of triethylamine were added, and the mixture was brought back to room temperature and stirred for 30 minutes. Chloroform was added, followed by washing with water. The solvent was removed from the obtained organic layer to yield 0.0002 mol of Compound 2.
¹H-NMR (400 MHz, acetone-d6) : δ (ppm) = 8.36 (1H), 7.83 (1H), 7.22 (4H), 7.02 (2H), 7.02 (2H), 3.82 (8H), 1.37 (12H)

### <Example 3>

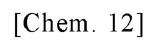

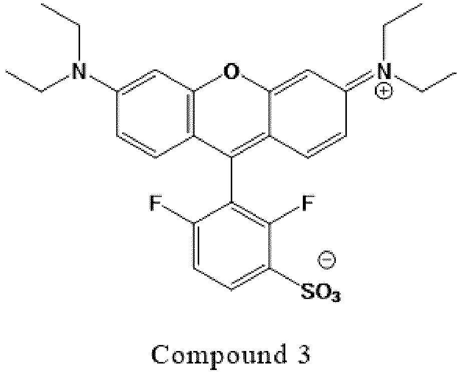

Compound 3 was obtained similarly to the method of Example 1, except that the starting material was changed from 2,6-dichlorobenzaldehyde to 2,6-difluorobenzaldehyde.
¹H-NMR (400 MHz, acetone-d6) : δ (ppm) = 8.16 (1H), 7.37 (2H), 7.20-7.30 (3H), 6.97 (2H), 3.78 (8H), 1.33 (12H)

### <Example 4>

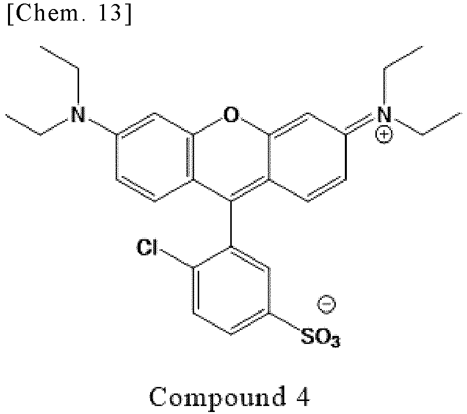

Compound 4 was obtained similarly to the method of Example 1, except that the starting material was changed from 2,6-dichlorobenzaldehyde to 2-chlorobenzaldehyde.
¹H-NMR (400 MHz, acetone-d6) : δ (ppm) = 7.98 (1H), 7.75 (1H), 7.64 (1H), 7.20 (4H), 6.96 (2H), 3.77 (8H), 1.32 (12H)

### <Comparative Example 1>

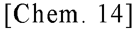

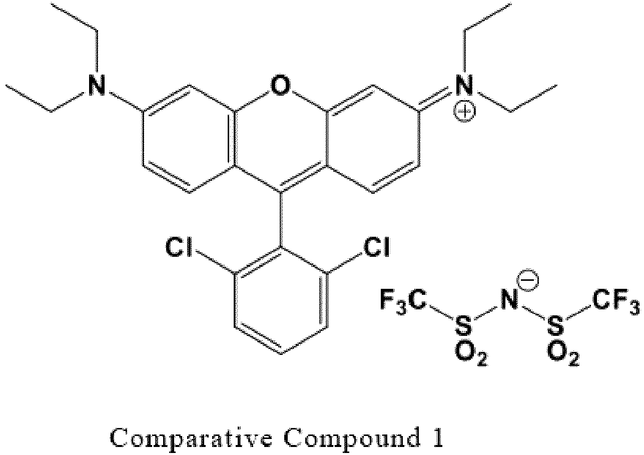

To 0.04 mol of 2,6-dichlorobenzaldehyde, 0.08 mol of diethylaminophenol and 0.36 mol of sulfuric acid were mixed. The mixture was stirred at 90 °C for 17 hours, followed by stirring at 120 °C for 1 hour. After being cooled to room temperature, the reaction solution was slowly added dropwise to an aqueous sodium hydroxide solution, and the resulting solid (49 g) obtained through filtration was mixed with 0.17 mol of dilute hydrochloric acid and 0.07 mol of a 35-mass% iron chloride solution. The mixture was stirred at 80 °C for 3 hours. After the reaction solution was cooled to room temperature, it was washed with toluene. The aqueous phase was neutralized with a sodium bicarbonate aqueous solution, and then toluene and 0.09 mol of potassium bis(trifluoromethanesulfonyl)imide were added and extraction was performed. The resulting organic layer was washed with water and the solvent was then removed. The residue was purified by silica gel column chromatography (developing solvent: ethyl acetate:chloroform = 90:10) to yield 2.4 g (0.01 mol) of Comparative Compound 1.
¹H-NMR (400 MHz, CDCl₃) : δ (ppm) = 7.60-7.50 (3H), 7.07 (6H), 6.97 (2H), 6.88 (2H), 3.64 (8H), 1.35 (12H)

The measurement results for each compound in Examples 1 to 4 and Comparative Example 1 are summarized below.

**[Table 1]**

| | | Example 1 | Example 2 | Example 3 | Example 4 | Comp. Example 1 |
|---|---|---|---|---|---|---|
| ΔE*ab | Held at 230 °C for 30 min. | 2.5 | 2.3 | 3.5 | 3.2 | 6.1 |
| | Held at 230 °C for 60 min. | 3.0 | 3.1 | 5.0 | 4.4 | 7.0 |
| | Held at 230 °C for 120 min. | 4.4 | 5.6 | 7.3 | 7.0 | 7.8 |

The color differences of chromatic values before and after holding at 230 °C (ΔE*_{ab}) in Examples 1 and 4 were smaller than those of Comparative Example 1, indicating improved heat resistance.

### INDUSTRIAL APPLICABILITY

The xanthene compound of the present disclosure has excellent heat resistance. It can be used in the production of a variety of paints, water-based or oil-based inks, or the like, and is also useful as a functional dye for recording materials or the like.

## Claims

1. A xanthene compound represented by the following formula (1): 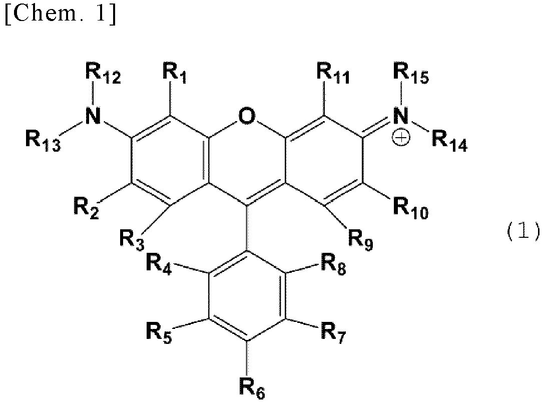 (where:
R₁ to R₃ and R₉ to R₁₁ are each independently a hydrogen atom, a halogen atom, a cyano group, a nitro group, a hydroxy group, a sulfone group, an alkyl group having 1 to 8 carbon atoms, a cycloalkyl group having 3 to 8 carbon atoms, an alkoxy group having 1 to 8 carbon atoms, an acyl group having 1 to 21 carbon atoms, an aryl group having 6 to 20 carbon atoms, or a five- to eight-membered heteroaryl group having 1 to 3 heteroatoms selected from nitrogen, oxygen, and sulfur atoms, wherein the alkyl group, the cycloalkyl group, and the alkoxy group may be substituted with a halogen atom or an aryl group having 6 to 20 carbon atoms, and the aryl group and the heteroaryl group may be substituted with a halogen atom, a cyano group, a nitro group, or a hydroxyl group,
R₄ to R₈ are each independently a hydrogen atom, an organic group, a fluorine atom, a chlorine atom, or an anionic group, with the proviso that at least one of R₄ to R₈ is a fluorine or chlorine atom and at least one of R₄ to R₈ is an anionic group,
R₁₂ to R₁₅ are each independently a hydrogen atom, an alkyl group having 1 to 8 carbon atoms, an aryl group having 6 to 20 carbon atoms, or a five- to eight-membered heteroaryl group having 1 to 3 heteroatoms selected from nitrogen, oxygen, and sulfur atoms, wherein the alkyl group may be substituted with an aryl group having 6 to 20 carbon atoms,
R₁ and R₁₂, R₂ and R₁₃, R₁₁ and R₁₅, and R₁₀ and R₁₄ may each be linked to form a six-membered ring,
R₁₂ and R₁₃, and R₁₄ and R₁₅ may each be linked to form a three- to six-membered ring).

2. The xanthene compound according to claim 1, wherein the anionic group is -SO₃⁻ or -SO₂N⁻SO₂R_{f} (where Rf is an alkyl group having 1 to 4 carbon atoms in which at least two hydrogen atoms are substituted with fluorine atoms).

3. The xanthene compound according to claim 1, wherein at least one of R₄ and R₈ is a fluorine or chlorine atom.

4. The xanthene compound according to claim 3, wherein both of R₄ and R₈ are fluorine or chlorine atoms.

5. A method for producing the xanthene compound of the formula (1) according to any of claims 1 to 4, the method comprising:
reacting, in the presence of an acid, a compound represented by the formula (10): 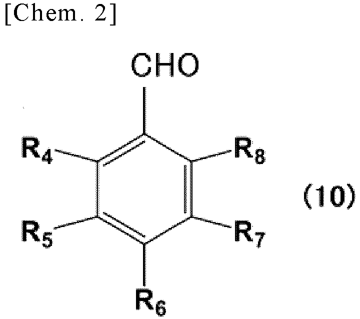 (where R₄ to R₈ are as defined in the formula (1) above), with compounds represented by the formulae (11) and (12): 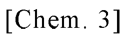 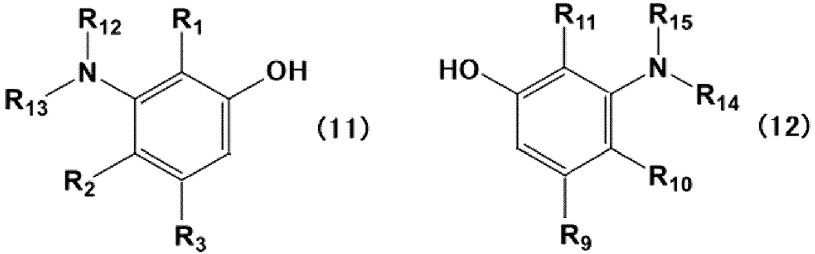 (where:
R₁ to R₃ and R₁₂ to R₁₃ are as defined in the formula (1) above, and
R₉ to R₁₁ and R₁₄ to R₁₅ are as defined in the formula (1) above); and
oxidizing a resultant compound represented by the formula (13): 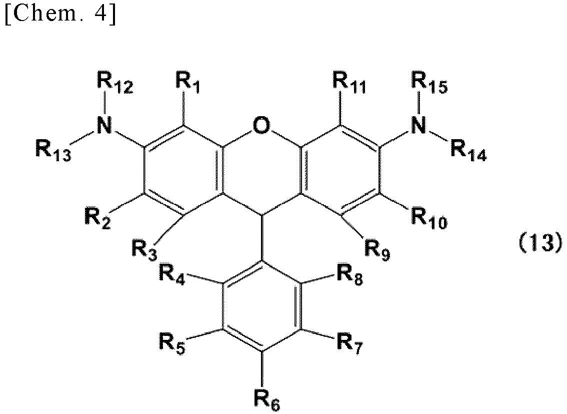 (wherein R₁ to R₁₅ are as defined in the formula (1) above).
